# EUROPÄISCHE PATENTSCHRIFT

(11) **EP 0 397 980 B1**
(45) Veröffentlichungstag und Bekanntmachung des Hinweises auf die Patenterteilung: **09.11.1994**
(21) Anmeldenummer: 90104643.3
(22) Anmeldetag: 12.03.1990
(51) Int. Cl.: A61B 17/22

(54) **Lithotriptor**
Lithotriptor
Lithotriteur

(30) Priorität: 17.05.1989 DE 3916093
(43) Veröffentlichungstag der Anmeldung: 22.11.1990
(73) Patentinhaber: Richard Wolf GmbH, 75438 Knittlingen (DE)
(72) Erfinder: Wurster, Helmut, D-7519 Oberderdingen (DE); Krauss, Werner, D-7134 Knittlingen (DE); Belikan, Thomas, D-7134 Knittlingen (DE)
(74) Vertreter: Wilcken, Thomas, Dipl.-Ing.

(56) Entgegenhaltungen:
- EP-A- 0 168 559
- EP-A- 0 169 311
- DE-U- 8 515 656
- FR-A- 2 610 818

## Beschreibung

Die Erfindung geht aus von einem Gerät zum Zerstören von Konkrementen oder Gewebe mittels Ultraschall-Stoßwellen gemäß dem Oberbegriff des Patentanspruchs 1. Ein derartiges Gerät ist in der DE-U-85 15 656 beschrieben.

Bei bekannten Lithotriptoren erfolgt die bei jeder Lithotripsie erforderliche, der Zerstörung eines Steines mittels Ultraschall-Stoßwellen vorausgehende Ortung des Steines durch ein Röntgensystem. Beispielhaft sei hier auch auf die aus dem DE-U-85 28 785 und dem DE-U-85 34 425 ersichtlichen Geräte hingewiesen.

Für eine derartige Ortung sind (mindestens) zwei Durchleuchtungsebenen notwendig. Üblicherweise verwendet man hierzu die sogenannte AP-Projektion und eine dieser gegenüber um 30° verschwenkte Projektion. Nach erfolgter Ortung des zu zerstörenden Steines werden dessen räumliche Koordinaten elektronisch ermittelt. Mittels einer komplizierten Elektronik und Mechanik wird hernach der Ultraschall-Stoßwellenwandler nun so geführt, daß sein Fokus in den durch die ermittelten räumlichen Koordinaten festgelegten Bereich zu liegen kommt. Erst dann ist die Applikation von Ultraschall-Stoßwellen möglich und medizinisch sinnvoll.

Bei den bekannten Lithotriptoren wird es als nachteilig empfunden, daß der Stoßwellenwandler nach der Ortung des zu zerstörenden Konkrementes mittels des Röntgensystems erst mit seinem Fokus auf das Konkrement ausgerichtet werden muß, um den Patienten nicht aus der Lage zu bringen, in welcher das Konkrement geortet worden ist. Darüberhinaus ist mit den bekannten Geräten eine bei vielen Indikationen angezeigte Ultraschall-Ortung des Konkrementes nicht möglich.

Aus der EP-A-0 168 559 ist ein Gerät zum räumlichen Orten und Positionieren von Konkrementen bekannt, das sowohl eine Röntgen-Ortungseinrichtung als auch eine Ultraschall-Ortungseinrichtung aufweist. Die Ortungssysteme dieser Einrichtung sind außerhalb des Wandlers angeordnet, ihre Strahlen bzw. Wellen verlaufen außerhalb des Stoßwellenfeldes, so daß das zu zerstörende Objekt zwar während der Stoßwellentherapie geortet werden kann, es jedoch nicht möglich ist festzustellen, ob der Weg zwischen Stoßwellenwandler und Konkrement frei von Hindernissen ist. So ist mit dieser Einrichtung nicht feststellbar, ob sich beispielsweise ein Knochen des Patienten im Stoßwellenweg befindet, der einerseits durch die Stoßwellen geschädigt werden und andererseits durch Abschattung eine Konkrementzerstörung behindern könnte.

Aus der FR-A-26 10 818 ist eine Einrichtung zur räumlichen Ortung und Zerstörung von körperinneren Objekten mit Ultraschall bekannt. Die Ortung des zu zerstörenden Objektes erfolgt bei dieser Einrichtung ausschließlich mit Ultraschall. Bekanntermaßen können schon aus physikalischen Gründen - die Ultraschallortung beruht auf Reflektion, während die Röntgenortung ein Durchdringungsverfahren darstellt - Röntgen-Ortungssysteme nicht durch Ultraschall-Ortungssysteme oder umgekehrt ersetzt werden, da in speziellen Anwendungsbereichen, beispielsweise wenn das zu ortende Konkrement sehr tief innerhalb des Körpers sitzt oder durch einen Knochen abgeschattet wird, eine Ultraschallortung nicht möglich ist.

Die der Erfindung zugrundeliegende Aufgabe besteht darin, ein Gerät der einleitend angeführten Art so zu verbessern, daß mit ihm eine Röntgenortung und eine Ultraschall-Ortung des zu zerstörenden Konkrementes oder Gewebes gleichzeitig oder jeweils für sich ohne Positionsänderung des Patienten möglich ist. Eine Röntgen-Echtzeitortung sowohl während der Stoßwellenapplikation als auch während der Verstellung der Ortungssysteme soll auch durchführbar sein.

Ausgehend von einem Gerät mit den Merkmalen des Oberbegriffes des Patentanspruches 1 wird diese Aufgabe durch die Merkmale des Kennzeichens dieses Anspruchs gelöst.

Die versetzte Anordnung des Röntgenstrahlers gegenüber der Wandlerachse hat den Vorteil, daß die Verschwenkbewegung des Röntgensystems ausgehend von der vertikalen Ausrichtung der Wandlerachse in einem geteilten Winkel um die Röntgenstrahlerachse ausgeführt werden kann. Ist die Röntgenstrahlerachse beispielsweise um 15° gegenüber der Wandlerachse versetzt, so bedarf es lediglich einer Verschwenkbewegung des Röntgensystems von ± 15° um die Röntgenstrahlerachse, um die bereits erwähnte AP-Projektion und die 30° Projektion zu erzeugen. Bei einer sogenannten Untertischanordnung der Einheit aus Röntgenstrahler, Ultraschall-Ortungswandler und Stoßwellenwandler wird durch den geteilten Verschwenkwinkel ein größerer Verfahrweg der Einheit in vertikaler Richtung erzielt, als dies bei Ausführung einer Verschwenkbewegung in einem ungeteilten Winkel möglich wäre, da die Einheit im letzteren Falle eher an die Unterseite des Behandlungstisches stoßen würde.

Gemäß einer vorteilhaften Weiterbildung ist das Iso-Zentrum für die Schwenkbewegung des Röntgensystems bzw. dessen Rahmens der erwähnte Fokus, wobei die gedachte Verlängerung der Schwenkachse des Rahmens bei jeder Schwenkstellung den Fokus schneidet. Hierdurch erübrigt sich ein Verfahren der Einheit aus dem Röntgenstrahler, Ultraschall-Ortungswandler und dem Stoßwellenwandler, da der Fokus des Stoßwellenwandlers bei einer Verschwenkbewegung des Röntgensystems zwecks Ortung des Konkrementes oder des Gewebes stets auf dieses ausgerichtet ist.

Im übrigen kann der Stoßwellenwandler ein piezoelektrischer, ein magnetostriktiver Wandler oder ein Wirbelstromwandler sein, wobei diese selbstfokussierend oder fokussierend über eine akustische Linse ausgebildet sind oder ein Reflektorsystem aufweisen. Aber auch ein Einsatz eines Wandlers mit einer Funkenentladungsstrecke ist durchaus möglich. Vorzugsweise ist der Wandler kalottenförmig ausgebildet, so daß die von ihm ausgesendeten Ultraschall-Stoßwellen von vornherein auf einen Fokus gerichtet sind.

Die Ultraschall-Ortungswandler können beispielsweise bekannte B-Scanner sein.

Da der Stoßwellenwandler mit den vorgenannten Ortungssystemen ausgestattet ist, kann eine Ortung des Konkrementes oder des Gewebes sowohl mit Hilfe des Röntgensystems als auch mittels des Ultraschall-Ortungswandlers vorgenommen werden. Auch eine Real-Time-Beobachtung des Zerstörungsvorganges des Konkrementes oder des Gewebes bei Applikation von Stoßwellen ist wahlweise mit einem der beiden Ortungssystemen oder gleichzeitig mit beiden Ortungssystemen möglich.

Ein Ultraschall-Ortungswandler ist vorteilhafterweise spiegelsymmetrisch zum Röntgenstrahler in bezug auf die Wandlerachse angeordnet. Ist der Röntgenstrahler beispielsweise um 15° gegenüber der Stoßwellenwandlerachse versetzt, so ist der Ultraschall-Ortungswandler diametral zu diesem ebenfalls um 15° gegenüber der Stoßwellenwandlerachse versetzt angeordnet. Durch diese Maßnahme wird erreicht, daß der Ortungswandler nicht oder nur im geringen Maße in die vom Röntgenstrahler ausgehende Röntgenstrahlkeule hineinragt.

Wenn bei dem erfindungsgemäßen Lithotriptor die vom Stoßwellenwandler erzeugten Stoßwellen über eine Flüssigkeit als Ankopplungsmedium auf den Körper des Patienten übertragen werden, kann es für eine verbesserte Röntgenortung vorteilhaft sein, wenn auf dem Röntgenstrahler ein gasgefüllter und zur Umgebung hin abgeschlossener Tubus vorgesehen ist, dessen Querschnitt dem Querschnitt der vom Röntgenstrahler zu Ortungszwecken ausgesandten Röntgenstrahlerkeule entspricht. Dadurch wird einer Dämpfung der Röntgenstrahlung im Ankopplungsmedium entgegengewirkt, dadurch, daß der Tubus das Ankopplungsmedium auf dem Wege der Röntgenstrahlung vom Röntgenstrahler zum Patientenkörper verdrängt.

Es ist vorteilhaft, wenn der Tubus etwa genausoweit in den Stoßwellenwandler hineinragt wie ein Ultraschall-Ortungswandler.

In einer weiteren Ausführungsform ist der Tubus entlang der Achse des Röntgenstrahlers verfahrbar. Hierdurch ist es möglich, ein optimales Verhältnis zwischen einerseits einer guten Röntgenbildqualität und andererseits einer möglichst geringen Abschattung des Stoßwellenfeldes zu erzielen.

Eine weitere vorteilhafte Weiterbildung dieser Ausführungsform des Lithotriptors ist dadurch gegeben, daß das dem Fokus zugewandte Ende des Tubus mit einem mit Gas füllbaren Ballon abgeschlossen sein kann. Hierdurch kann bei der vorerwähnten Ausführungsform zwischen dem Tubusende und dem Patientenkörper gegebenenfalls noch befindliches Ankopplungsmedium vollständig verdrängt werden. Zum Zwecke einer optimalen Röntgenortung kann der Ballon mittels einer Druckpumpe über eine geeignete Leitung mit Gas aufgepumpt werden. Nach erfolgter Ortung kann der Ballon mittels einer Vakuumpumpe evakuiert werden, so daß die Stoßwellen vom Stoßwellenwandler während der Therapie wieder optimal durch das Ankopplungsmedium an den Patientenkörper angekoppelt werden. Um den Druck während des Aufblasens des Ballons im Ankopplungsmedium konstant zu halten, kann ein dem Volumen des Ballons stets entsprechendes Volumen der Ankopplungsflüssigkeit einem an sich bekannten Ausgleichsystem zugeführt werden. Während des Ablassens des Gases aus dem Ballon über die erwähnte Vakuumpumpe kann die zuvor dem Ausgleichssystem zugeführte Ankopplungsflüssigkeit wieder zurückgeführt werden.

Eine vorteilhafte Möglichkeit zur Positionierung des Fokus auf das Konkrement oder das zu zerstörende Gewebe ist gegeben, wenn der Behandlungstisch, auf dem der Patient ruht, in den Koordinaten X, Y und Z verfahrbar ist, wobei die Ortungssysteme ortsfest zu dem Wandler angeordnet und zusammen mit dem Wandler um dessen Fokus schwenkbar sind.

Im Falle der schon erwähnten Untertischanordnung weist die Platte des Behandlungstisches eine Öffnung zum Durchtritt der Röntgenstrahlen und der Ultraschallwellen auf. Der betreffende Körperteil, in welchem sich das Konkrement oder das zu zerstörende Gewebe befindet, ist dabei im Bereich der erwähnten Öffnung positioniert.

Es ist aber auch möglich, den erfindungsgemäßen Lithotriptor in einer sogenannten Übertischanordnung zu betreiben. Hierbei befindet sich der Bildverstärker des Röntgensystems unterhalb des Patienten, die Einheit aus Stoßwellenwandler, Röntgenstrahler und Ultraschall-Ortungswandler hingegen über dem Patienten. Dies bietet beispielsweise Vorteile für die Therapie zur Zerstörung von Gallenblasensteinen. In diesem Falle braucht die Platte des Behandlungstisches natürlich keine Öffnung aufzuweisen,woraus eine verbesserte Patientenlagerung resultiert. Es ist verständlich, daß die Platte des Behandlungstisches hierbei aus röntgenstrahlendurchlässigem Material, beispielsweise Plexiglas, bestehen sollte. Anderenfalls wäre eine Röntgenortung nicht möglich.

Schließlich kann vorgesehen sein, das Röntgensystem zusammen mit dem Stoßwellenwandler an dem schon erwähnten Rahmen anzuordnen, der mittels eines an einer vorzugsweise vertikalen Säule angreifenden Antriebes in vertikaler Richtung verfahrbar, um die Achse der Säule und um die Schwenkachse des Rahmens um einen Winkel verschwenkbar ist. Bei kleinen Verschwenkwinkeln, beispielsweise ± 15° um die Ruhelage wird es sich um den oben erwähnten geteilten Verschwenkwinkel zum Zwecke der Röntgenortung des Konkrementes bzw. Gewebes handeln. Bei einem Schwenkwinkel von 180° wird das System beispielsweise von einer Untertischanordnung der Einheit aus Stoßwellenwandler, Röntgenstrahler und Ultraschall-Ortungswandler in eine Übertischanordnung dieser Einheit gebracht. Hernach kann das System wiederum zu Ortungszwecken um kleine Winkel, beispielsweise wieder um ± 15°, geschwenkt werden.

Die Erfindung wird anhand einiger Ausführungsbeispiele näher erläutert: Hierbei zeigt:
- Figur 1: ein erstes Ausführungsbeispiel des Lithotriptors mit einen in den Koordinaten X, Y, und Z verfahrbaren Behandlungstisch,
- Figur 2: die Einheit aus einem Stoßwellenwandler, einem Röntgenstrahler und einem Ultraschall-Ortungswandler in einer Untertischanordnung und den Bildverstärker, wobei die Verschwenkbewegung zum Zwecke der Röntgenortung schematisch angedeutet ist,
- Figur 3: eine Ausführungsform der Einheit aus Stoßwellenwandler, Röntgenstrahler und UltraschallOrtungswandler,
- Figur 4: eine weitere Ausführungsform der Einheit aus Stoßwellenwandler, Röntgenstrahler und Ultraschall-Ortungswandler, Figur 5 eine weitere Ausführungsform des Lithotriptors, und
- Figur 6: eine noch weitere Ausführungsform des Lithotriptors.

Im Folgenden sind gleiche Teile mit denselben Bezugszeichen bezeichnet.

Die Einheit aus einem Stoßwellenwandler 1, dem Röntgenstrahler 2 und dem Ultraschall-Ortungswandler 4, welche weiter unten näher beschrieben wird, ist an einem freien Schenkel eines U-förmigen Rahmens 7 angeordnet. Am anderen Schenkel dieses Rahmens 7 ist dieser Einheit gegenüberliegend ein Bildverstärker 3 befestigt. Der U-förmige Rahmen 7 weist eine Schwenkachse 6 auf, um welche der Rahmen 7 mittels eines Antriebes, wie weiter unten ausgeführt, um einen Winkel α verschwenkbar ist. Der Lithotriptor weist einen Behandlungstisch, bestehend aus der Platte 8 und dem Tischständer 14 auf. Ein im Tischständer 14 untergebrachter Antrieb (nicht dargestellt) vermag die Platte 8 in den Koordinaten X, Y und Z zu verfahren, so daß der Fokus 5 des Stoßwellenwandlers 1 exakt auf das im Körper eines auf der Platte 8 zu liegen kommenden Patients befindliche zu zerstörende Konkrement oder Gewebe ausrichtbar ist.

In der Platte 8 befindet sich eine Öffnung 12, in welcher der das zu zerstörende Konkrement oder Gewebe enthaltende Körperteil eines Patienten gelagert wird.

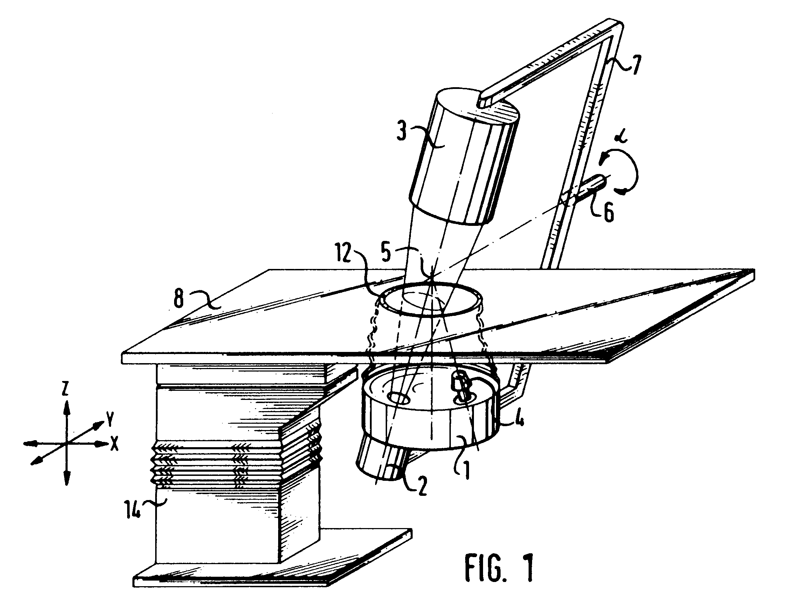

Wie schon aus Figur 1 ersichtlich, ist der Röntgenstrahler 2 und der Ultraschall-Ortungswandler 4 in dem Stoßwellenwandler 1 angeordnet und gegenüber der Stoßwellenwandlerachse derart versetzt, daß sich die Wandlerachse, die Röntgenstrahlerachse und die Achse des Ultraschall-Ortungswandlers 4 im Fokus 5 des Stoßwellenwandlers 1 schneiden. Im dargestellten Ausführungsbeispiel ist der Fokus 5 das Iso-Zentrum für die Schwenkbewegung des Röntgensystems 2,3 bzw. dessen Rahmens 7. Die gedachte Verlängerung der Schwenkachse 6 des Rahmens 7 schneidet dabei bei jeder Schwenkstellung den Fokus, wie dies schematisch in Figur 1 dargestellt ist.

Bei dem dargestellten Lithotriptor ist der UltraschallOrtungswandler 4 spiegelsymmetrisch zum Röntgenstrahler 2 in bezug auf die Stoßwellenwandlerachse versetzt angeordnet.

Der Stoßwellenwandler 1 und die mit ihm verbudnenen Teile lassen sich aus der dargestellten Untertischanordnung durch Verschwenkung des Rahmens um einen Winkel α = 180° in eine sogenannte Übertischanordnung bringen. Wenn auch die Untertischanordnung eine nur geringe Belastung des Bedienungspersonals durch Röntgenstrahlung ermöglicht und ihr im allgemeinen der Vorzug zu geben sein wird, kann es bei einigen Therapien angezeigt sein, die Übertischanordnung zu verwenden.

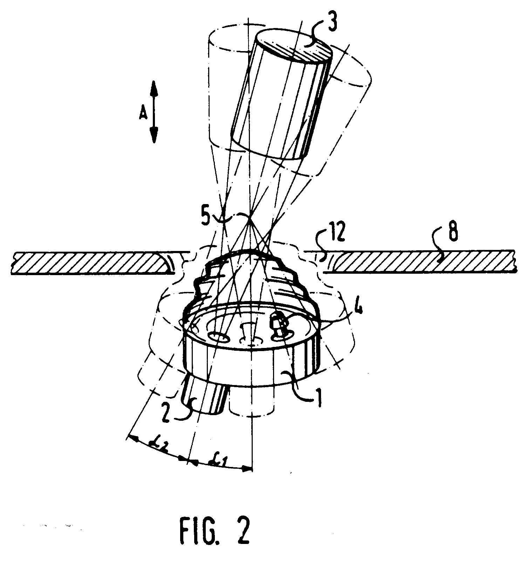

Der Vorteil der versetzten Anordnung des Röntgenstrahlers 2 im Stoßwellenwandler 1 wird anhand der Figur 2 erläutert.

Figur 2 zeigt die Einheit aus Stoßwellenwandler 1, Röntgenstrahler 2 und Ultraschall-Ortungswandler 4 in einer Untertischanordnung unterhalb der Platte 8 sowie den dem Röntgenstrahler 2 gegenüberliegend am Rahmen (hier nicht dargestellt) angeordneten Bildverstärker.

Im dargestellten Ausführungsbeispiel ist der Röntgenstrahler 2 in der Kalotte des Stoßwellenwandlers 1 derart versetzt angeordnet, daß die Achse des Röntgenstrahlers 2 und die Achse des Stoßwellenwandlers 1 einen Winkel von 15° einschließen. Spiegelsymmetrisch zum Röntgenstrahler 2 ist diesem diametral gegenüberliegend der Ultraschall-Ortungswandler 4 angeordnet.

Wie schon oben ausgeführt, ist es bei der Röntgenortung des zu zerstörenden Konkrementes oder Gewebes erforderlich, den Patientenkörper in zwei Ebenen zu durchstrahlen. Überlicherweise wird dies mit einer sogenannten AP-Projektion, bei welcher die Röntgenstrahlen den Patientenkörper senkrecht durchstrahlen, und mit einer dieser gegenüber um 30° verschwenkten Projektionen vorgenommen.

Um dies mit dem erfindungsgemäßen System zu erreichen, bedarf es für die AP-Projektion einer Verschwenkung lediglich um einen Winkel α₁ von 15° und für die 30°-Projektion lediglich um einen Winkel α₂ von 15° in die andere Richtung, jeweils von der Ruhelage aus gesehen, bei welcher die Achse des Stoßwellenwandlers 1 senkrecht steht. Hierdurch wird ein größtmöglicher Verfahrweg in Richtung des Doppelpfeiles A erreicht, auf welchem das System gegebenenfalls so bewegt werden muß, daß der Fokus 5 mit dem zu zerstörenden Konkrement oder Gewebe koinzident ist.

Der Vorteil der versetzten Anordnung des Röntgenstrahlers im Stoßwellenwandler 1 ergibt sich in anschaulicher Weise aus der folgenden Betrachtung: Würde der Röntgenstrahler beispielsweise zentral im Stoßwellenwandler 1 angeordnet sein, so würde der Verfahrweg des Systems auf die Platte 8 hin erheblich dadurch begrenzt werden, daß der Stoßwellenwandler 1 bei der Ausführung einer Verschwenkbewegung um einen (ungeteilten) Winkel von 30° an die Unterseite der Platte 8 stoßen würde.

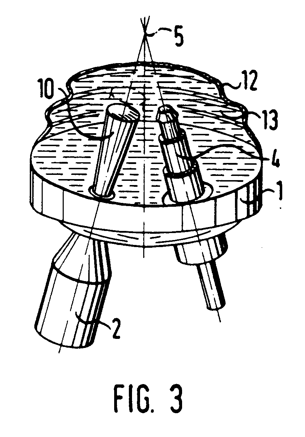

Figur 3 zeigt eine weitere vorteilhafte Ausführungsform der Einheit aus Stoßwellenwandler 1, Röntgenstrahler 2 und Ultraschall-Ortungswandler 4. Zur besseren Ankopplung des Stoßwellenwandlers 1 an den Körper des Patienten ist die Kalotte des Wandlers 1 mit einer Flüssigkeit als Ankopplungsmedium 13, bspw. Wasser, gefüllt und nach außen hin mit einer Membran 12 abgeschlossen.

Auf den Röntgenstrahler 2 ist vorliegend ein gasgefüllter und zur Umgebung hin abgeschlossener Tubus 10 angeordnet. Der Querschnitt des Tubus 10 ist dem Querschnitt der vom Röntgenstrahler 2 zu Ortungszwecken ausgesandten Röntgenstrahlkeule angepaßt. Der Tubus 10 verdrängt das Ankopplungsmedium 13 dauerhaft. Hierdurch wird die Dämpfung der Röntgenstrahlung im Ankopplungsmedium im Sinne einer besseren Röntgenortung herabgesetzt. Wie schematisch angedeutet, ist der Tubus 10 entlang der Achse des Röntgenstrahlers 2 verfahrbar.

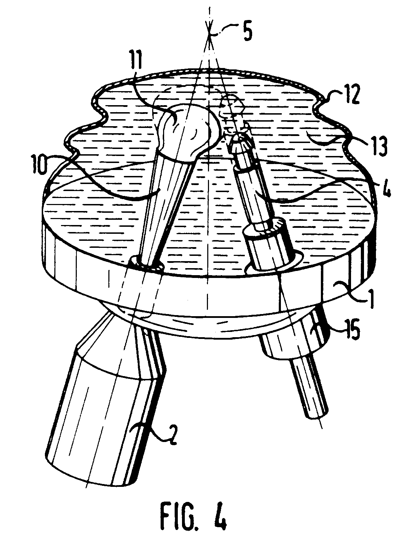

Gemäß dem Ausführungsbeispiel der Figur 4 ist das dem Fokus 5 zugewandte Ende des Tubus 10 mit einem Ballon 11 abgeschlossen. Der Ballon 11 ist mit Hilfe einer (nicht dargestellten) Pneumatik mit Gas füllbar bzw. evakuierbar. Wenn zu Zwecken der Röntgenortung der Weg der Röntgenstrahlen durch das Ankopplungsmedium 13 weiter verkürzt werden soll, so wird der Ballon 11 mit Gas aufgeblasen. Das dabei verdrängte Ankopplungsmedium 13 wird in einem (nicht dargestellten) Ausgleichssystem aufgefangen, damit der Druck innerhalb des von der Membran 12 eingeschlossenen Raums konstant bleibt. Zur Einleitung der Applikation der Ultraschall-Stoßwellen wird der Ballon 11 durch die (nicht dargestellte) Pneumatik evakuiert. Gleichzeitig wird das zuvor im (nicht dargestellten) Ausgleichssystem aufgefangene Ankopplungsmedium 13 wieder zurück geführt.Hierdurch wird die einwandfreie Ankopplung des Stoßwellenwandlers 1 an den Patientenkörper während der Therapie gewährleistet.

Im gezeigten Ausführungsbeispiel ist im übrigen der Ultraschall-Ortungswandler 4 mit einem Scannergetriebe 15 verbunden, welches ihn längs seiner Längsachse verfahren und um seine Achse zu drehen vermag.

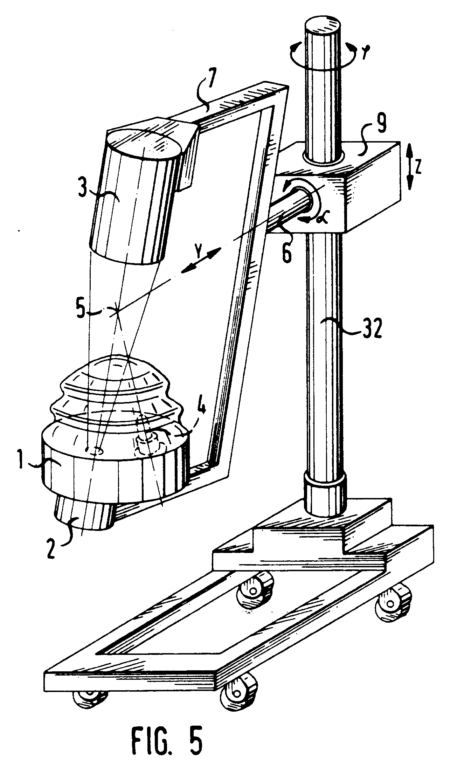

Figur 5 zeigt eine weitere Ausführungsform des Lithotriptors. Hier ist die Einheit aus dem Stoßwellenwandler 1, Röntgenstrahler 2 und Ultraschall-Ortungswandler 4 sowie der Bildverstärker 3 an einem Rahmen 7 angeordnet, der über die Schwenkachse 6 mittels eines an einer vertikalen Säule 32 angreifenden Antriebes 9 in vertikaler Richtung verfahrbar ist. Darüberhinaus gestattet der Antrieb 9 die Ausführung einer Bewegung in Richtung der Koordinate Y und eine Verschwenkbewegung um die Achse der Säule 32 um einen Winkel φ sowie ein Verschwenken um die Schwenkachse 6 des Rahmens 7 um einen Winkel α.

Der Antrieb 9 gestattet im übrigen Verschwenkbewegungen um kleine Winkel α, beispielsweise um ± 15°, zu Ortungszwecken, aber auch die Verschwenkung um große Winkel, beispielsweise um 180°, zum Zwecke der Überführung des Systems von einer Untertischanordnung hin zu einer Übertischanordnung.

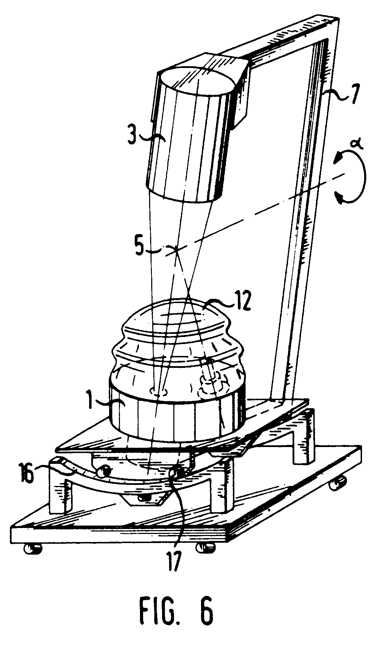

Figur 6 schließlich zeigt eine noch weitere Ausführungsform des Lithotriptors.

Hierbei ist die Einheit aus Stoßwellenwandler 1, Röntgenstrahler 2 und Ultraschall-Ortungswandler 4 gemeinsam mit dem Bildverstärker 3 konfokal um den Fokus 5 auf der bogenförmig gekrümmten Führung 16 verschwenkbar und den Winkel α (in beiden Richtungen). Mit dem System in geeigneter Weise verbundene Rollen 17 oder dergleichen rollen auf oder an der Führung 16 ab, so daß eine Verschwenkbewegung um den Winkel α, beispielsweise um ± 15°, zu Ortungszwecken ausgeführt werden kann.

## Patentansprüche

1. Gerät zum Zerstören von Konkrementen oder Gewebe mittels Ultraschall-Stoßwellen mit einem Wandler (1) zur Erzeugung von fokussierten Ultraschall-Stoßwellen, wobei der Fokus (5) auf das jeweils zu zerstörende Konkrement oder Gewebe ausrichtbar ist, und mit mindestens einem bildgebenden diagnostischen Röntgensystem (2,3) zur Ortung des Konkrementes oder Gewebes, dessen Röntgenstrahler (2) und dessen dem Röntgenstrahler gegenüberliegender Bildverstärker (3) an einem Rahmen (7) angeordnet sind, der zwecks Ortung in verschiedenen Bildebenen um eine Achse (6) verschwenkbar ist, dadurch gekennzeichnet, daß jeweils mindestens ein Röntgenstrahler (2) und ein Ultraschall-Ortungswandler (4) in dem Wandler (1) angeordnet und gegenüber der Wandlerachse derart versetzt sind, daß sich die Wandlerachse, die Röntgenstrahlerachsen und die Achsen der Ultraschall-Ortungswandler im Fokus (5) des Wandlers (1) schneiden.

2. Gerät nach Anspruch 1, dadurch gekennzeichnet, daß das Iso-Zentrum für die Schwenkbewegung des Röntgensystems (2,3) bzw. dessen Rahmen (7) der Fokus (5) des Wandlers ist, wobei die gedachte Verlängerung der Schwenkachse (6) des Rahmens (7) bei jeder Schwenkstellung den Fokus (5) schneidet.

3. Gerät nach Anspruch 1 oder 2, dadurch gekennzeichnet, daß ein Ultraschall-Ortungswandler (4) spiegelsymmetrisch zum Röntgenstrahler (2) in Bezug auf die Wandlerachse angeordnet ist.

4. Gerät nach einem der Ansprüche 1 bis 3, dadurch gekennzeichnet, daß auf dem Röntgenstrahler (2) jeweils ein gasgefüllter und zur Umgebung hin abgeschlossener Tubus (10) vorgesehen ist, dessen Querschnitt mindestens dem Querschnitt der vom Röntgenstrahler zu Ortungszwecken ausgesandten Röntgenstrahlkeule entspricht.

5. Gerät nach Anspruch 4, dadurch gekennzeichnet, daß der Tubus (10) zu Ortungszwecken entlang der Achse des Röntgenstrahlers (2) verfahrbar ist.

6. Gerät nach Anspruch 4 oder 5, dadurch gekennzeichnet, daß das jeweils mit dem Fokus (5) zugewandte Ende des Tubus (10) mit einem mit Gas füllbaren Ballon (11) abgeschlossen ist.

7. Gerät nach einem der Ansprüche 1 bis 6, dadurch gekennzeichnet, daß zur Positionierung des Fokus (5) auf das zu zerstörende Konkrement oder Gewebe ein in den Koordinaten X, Y, Z verfahrbarer Behandlungstisch (8,14) vorgesehen ist, auf dem der zu behandelnde Patient zum Liegen kommt, und daß die Ortungssysteme (2, 3, 4) ortsfest zu dem Wandler (1) und zusammen mit dem Wandler um dessen Fokus (5) schwenkbar angeordnet sind.

8. Gerät nach einem der Ansprüche 1 bis 7, dadurch gekennzeichnet, daß die Ortungssysteme (2, 3, 4) zusammen mit dem Wandler (1) an dem Rahmen (7) angeordnet sind, der mittels eines an einer Säule (32) angreifenden Antriebs (9) in Richtung der Koordinaten Y und Z verfahrbar, um die Achse der Säule (32) um einen Winkel φ und um die Schwenkachse (6) des Rahmens (7) um einen Winkel α verschwenkbar ist.

9. Gerät nach einem der Ansprüche 1 bis 8, dadurch gekennzeichnet, daß die Ortungssysteme (2, 3, 4) mit dem Wandler (1) an dem Rahmen (7) angeordnet und gemeinsam auf einer bogenförmig gekrümmten Führung (16) konfokal um den Fokus (5) verschwenkbar sind.

## Claims

1. Apparatus for destroying concretions or tissue by means of ultrasound shock waves having a transducer (1) for producing focused ultrasound shock waves, the focal point (5) being alignable with the particular concretion or tissue to be destroyed, and having at least one image-forming diagnostic X-ray system (2, 3) for locating the concretion or tissue, the X-ray emitter (2) of which and the image intensifier (3) of which opposite the X-ray emitter being arranged on a frame (7), which can be pivoted about an axis (6) for the purpose of location in different image planes, characterised in that in each case at least one X-ray emitter (2) and one ultrasound location transducer (4) are arranged in the transducer (1) and offset with respect to the transducer axis, such that the transducer axis, the X-ray emitter axes and the axes of the ultrasound location transducer intersect in the focal point (5) of the transducer (1).

2. Apparatus according to claim 1, characterised in that the isocentre for the pivoting movement of the X-ray system (2, 3) or its frame (7) is the focal point (5) of the transducer, the intended extension of the pivoting axis (6) of the frame (7) intersecting the focal point (5) in each pivoting position.

3. Apparatus according to claim 1 or 2, characterised in that an ultrasound location transducer (4) is arranged in mirror symmetry to the X-ray emitter (2) with respect to the transducer axis.

4. Apparatus according to one of claims 1 to 3, characterised in that in each case a gas-filled cone (10) sealed from the surroundings is provided on the X-ray emitter (2), the cross-section of the cone (10) corresponding at least to the cross-section of the X-ray beam radiated by the X-ray emitter for location purposes.

5. Apparatus according to claim 4, characterised in that the cone (10) can be moved along the axis of the X-ray emitter (2) for location purposes.

6. Apparatus according to claim 4 or 5, characterised in that the particular end of the cone (10) facing the focal point (5) is sealed by a balloon (11) which can be filled with gas.

7. Apparatus according to one of claims 1 to 6, characterised in that a treatment table (8, 14) which can be moved in the coordinates X, Y, Z is provided for positioning the focal point (5) on the concretion or tissue to be destroyed, on which treatment table (8, 14) the patient to be treated comes to rest, and in that the location systems (2, 3, 4) are arranged to be fixed with respect to the transducer (1) and together with the transducer to be pivotable about its focal point (5).

8. Apparatus according to one of claims 1 to 7, characterised in that the location systems (2, 3, 4) together with the transducer (1) are arranged on the frame (7), which can be moved in the direction of coordinates Y and Z by means of a drive (9) acting on a column (32), can be pivoted about the axis of the column (32) by an angle φ and about the pivoting axis (6) of the frame (7) by an angle α.

9. Apparatus according to one of claims 1 to 8, characterised in that the location systems (2, 3, 4) and the transducer (1) are arranged on the frame (7) and together are pivotable confocally about the focal point (5) on an arc-shaped curved guide (16).

## Revendications

1. Appareil de destruction de calculs ou de tissus à l'aide d'ondes de choc ultrasoniques, comportant un transducteur (1) pour produire des ondes de choc ultrasoniques focalisées, le foyer (5) pouvant être aligné sur respectivement le calcul ou le tissu à détruire, et au moins un système radiographique de diagnostic formateur d'image (2, 3) pour repérer le calcul ou le tissu, dont le générateur de rayons X (2) et son amplificateur d'image (3) en regard du générateur de rayons X sont agencés sur un cadre (7), qui peut pivoter autour d'un axe (6) dans divers plans d'image à des fins de repérage, caractérisé en ce que respectivement au moins un générateur de rayons X (2) et un transducteur de repérage ultrasonique (4) sont agencés dans le transducteur (1) et sont décalés vis-à-vis de l'axe du transducteur de telle sorte que l'axe du transducteur, les axes du générateur de rayons X et les axes du transducteur de repérage ultrasonique se coupent au foyer (5) du transducteur (1).

2. Appareil selon la revendication 1, caractérisé en ce que l'isocentre de pivotement du système radiographique (2, 3) ou de son cadre (7) est le foyer (5) du transducteur, le prolongement imaginaire de l'axe de pivotement (6) du cadre (7) coupant le foyer (5) dans chaque position de pivotement.

3. Appareil selon la revendication 1 ou 2, caractérisé en ce qu'un transducteur de repérage ultrasonique (4) est agencé en symétrie de miroir vis-à-vis du générateur de rayons X (2) par rapport à l'axe du transducteur.

4. Appareil selon les revendications 1 à 3, caractérisé en ce qu'il est prévu sur le générateur de rayons X (2) respectivement un tube rempli de gaz fermé vis-à-vis de l'extérieur (10), dont la section transversale correspond au moins à la section transversale du faisceau de rayons X émis par le générateur de rayons X à des fins de repérage.

5. Appareil selon la revendication 4, caractérisé en ce que le tube (10) peut être déplacé à des fins de repérage le long de l'axe du générateur de rayons X (2).

6. Appareil selon la revendication 4 ou 5, caractérisé en ce que l'extrémité du tube (10) respectivement tournée vers le foyer (5) est fermée par un ballon (11) que l'on peut remplir de gaz.

7. Appareil selon l'une quelconque des revendications 1 à 6, caractérisé en ce que, pour positionner le foyer (5) sur le calcul ou le tissu à détruire, il est prévu une table de traitement (8, 14) qui peut être déplacée selon les coordonnées X, Y et Z, table sur laquelle le patient à traiter est étendu, et en ce que les systèmes de repérage (2, 3, 4) sont agencés fixes par rapport au transducteur (1) et peuvent pivoter conjointement avec le transducteur autour de son foyer (5).

8. Appareil selon l'une quelconque des revendications 1 à 7, caractérisé en ce que les systèmes de repérage (2, 3, 4) sont agencés conjointement avec le transducteur (1) sur le cadre (7), qui peut être déplacé à l'aide d'un système d'entraînement (9) coopérant avec une colonne (32) dans la direction des coordonnées Y et Z et peut pivoter autour de l'axe de la colonne (32) d'un angle φ et autour de l'axe de pivotement (6) du cadre (7) d'un angle α.

9. Appareil selon l'une quelconque des revendications 1 à 8, caractérisé en ce que les systèmes de repérage (2, 3, 4) sont agencés avec le transducteur (1) sur le cadre (7) et peuvent pivoter conjointement sur un guide cintré en arc (16) en foyer commun autour du foyer (5).
